# EUROPEAN PATENT SPECIFICATION

(11) **EP 0 438 368 B1**
(45) Date of publication and mention of the grant of the patent: **02.02.1994**
(21) Application number: 91500005.3
(22) Date of filing: 15.01.1991
(51) Int. Cl.: A61M 5/32, A61M 5/50

(54) **Disposable hypodermic syringe**
Wegwerfbare Injektionsspritze
Seringue hypodermique à usage unique

(30) Priority: 17.01.1990 ES 9000126
(43) Date of publication of application: 24.07.1991
(73) Proprietor: Caralt Batlle, Jaime, E-08230 Matapedrera (ES)
(72) Inventor: Caralt Batlle, Jaime, E-08230 Matapedrera (ES)
(74) Representative: Toro Gordillo, Ignacio Maria

(56) References cited:
- WO-A-89/00435
- US-A- 3 306 290
- US-A- 4 692 156
- US-A- 4 747 831
- US-A- 4 838 869

## Description

This invention relates to a disposable hypodermic syringe of the type which is specified in the preamble of claim 1.

### Technical background

The use of disposable hypodermic syringes which are sold in sterilized and sealed packages of one unit each and which are broken and thrown away after they are used once, is well known.

However, although these syringes are made and sold to be used only once, the truth is that their structural characteristics allow their repeated use without any difficulty from the mechanical point of view, although this gives rise to obvious and very serious problems in the area of health.

These problems have become more acute recently, since the appearance of AIDS, which eventually causes the death of the patient, and for which there is still no known cure or vaccination which will stop it from spreading.

Although there have been attemps to come up with disposable syringes to avoid their repeated use, the only ones which really work are those which, automatically and without any subsequent handling by the user, prevent any further use.

US-A-4838869, which corresponds to the preamble of claim 1, discloses a disposable hypodermic syringe having means for retaining the needle in an operative position thereof, and releasing means on the piston end releasing the needle when the end of the piston run is attained, so that a spring pushing constantly the needle toward the inside forces the needle to move inwards when these retaining devices are released to conceal the needle in the hollow portion of the piston.

However, this known device does not provide means to hold the needle in that concealing position inwards, excepting those constituted by the spring itself.

### Description of the invention

According to the present invention, the posed problem is solved in a simple and efficient way by a hypodermic syringe having the characteristics recited in claim 1.

Accoring to a further characteristic of this invention, the interlocking retention appendages in the front part of the tube are made up of a plurality of flexible tongues which rise from the wall of the tube at a slanting angle, facing foward and inward, and which are set so that they become fitted into a ring-shaped external groove made around the outside of the needle's head, from where they are removed as they are bent by means of the pushing action of the appendages of the obturating discoid part previously mentioned.

Considering anther characteristic of the invention, the closing device of the piston cavity is constituted by a discoidal button, with its edge pressure-fitted into a ring shaped groove made around the mouth of the piston cavity. This discoid button has a protuberance in its front part, which constitutes said irreversible joining devices, and which has an external stepped protruding edge around it, which will become pressure-fitted into a countersinked hole in the needle head, from where it is impossible to be extracted due to the opposing action of the complementary joining devices, which in turn, consist on an intend step.

Another feature of this invention is that piston-retaining devices are arranged at the mouth of the tube cavity which make the total extraction of the piston from the tube impossible.

### Brief description of the drawings

The enclosed drawing show, by way of an example not to be considered as exclusive, a way in which the hypodermic syringe subject of this invention, can be made.

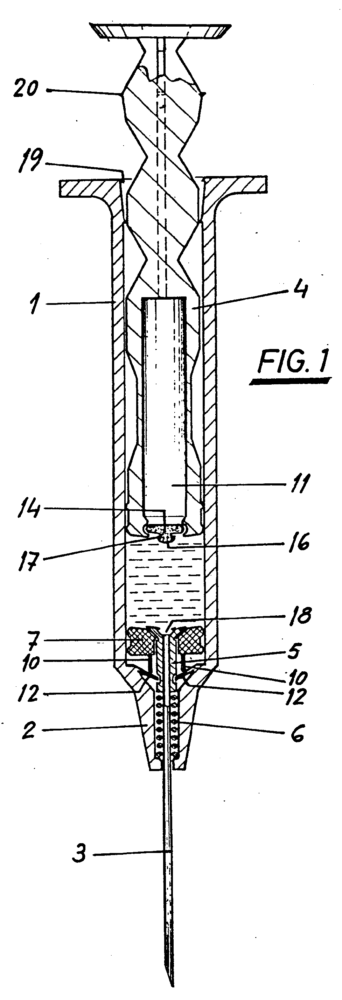

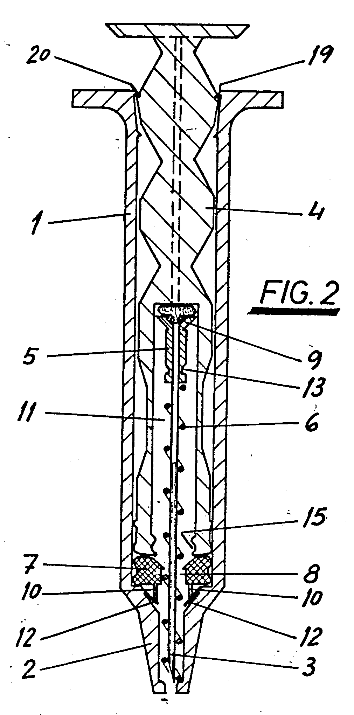

Figure 1 is the cross section of an elevational view of a syringe prior of its use; and Figure 2 is a similar view of the one shown in Figure 1, but once the syringe has been rendered useless.

### The optimum method to make this invention

The enclosed drawing show that the disposable hypodermic syringe, which is the subject of this invention, is comprised of a tube 1, having its front part 2 narrowed and adapted to receive a hypodermic needle 3, and a piston 4 capable of sliding through the inside of the tube 1.

Obturating devices 7 held in an operative position by the head 5 of the needle 3 are laid across the front inside part of the tube 1.

Said head 5 of the needle is pressed by a spring 6, preferentially constituted by a helicoidal compression ring, which tends to push it towards the inside of the tube 1, and is held by means of interlocking retention devices located in the inside part of the tube 1.

At the front end of piston 4, there is a closing device of the piston cavity 11, having irreversible joining devices located at the head 5 of the needle 3.

Said obturating devices are composed of a discoid part 7 of a slightly flexible material, its edge being in contact with the inside wall of the front end of the tube 1, so accoplishing a tight sealing with the latter.

The discoid part 7 has a central countersunk hole 8 to receive the conical end 9 of the head 5 of the needle 3 which passes through it, so accomplising also a tight sealing with said conical end 9.

The discoid part 7 has also, at its front side, appendages 10 adapted to operate on the interlocking retention devices in the inside front part of tube 1, as the discoid part 7 is pushed forward by the front part of the piston 4, when it completes its operative run.

At this stage, the discoid part 7 and the needle 3 with it, moves slightly foward as they are pushed by piston 4. Said appendages 10, in turn, push the retention devices, thereby releasing the needle 3, which is introduced into the cavity 11 of piston 4, through the action of spring 6.

Said interlocking retention devices located at the front part of the tube 1 are made up of a plurality of flexible tongues 12, arising from the wall of the tube 1 at the slanting angle, facing foward and inward, and which are set so that they become fitted into a ring shaped external groove 13 made around the outside of the head 5 of needle 3, from where they are removed as they are bent by means of the pushing action of the appendages 10 of the obturating discoid part 7 previously mentioned.

Said closing device of the cavity 11 of the piston 4 is constituted by a discoid button 14 having its edge pressure-fitted into a ring shaped groove 15 made around the mouth of said cavity 11.

Said discoid button has a protuberance 16, constituting said irreversible joining devices, and which has an external stepped protruding edge around it 17, to be pressure-fitted into a countersunk hole 18 in the head 5 of needle 3 constituting said complementary joining device, from where it is impossible to be extracted due to the opposing action of said complementary interlocking retention devices, which in turn, consist of an indented step.

At the mouth of the cavity of tube 1, there are arranged retaining means for the piston 4, which are preferentially constituted by joining ribs 19 and 20, which make the total extraction of the piston from the tube 1 impossible.

## Claims

1. Disposable hypodermic syringe, of the type which is rendered unusable after it has been used once, comprising a tube (1) with its front part narrowed and adapted to receive a hypodermic needle (3), the head (5) of which is pressed by a spring (6) tending to push it towards the inside of the tube (1); a hollow piston (4), which slides through the inside part of the tube (1); interlocking retention devices (12) placed in the front inside part of the tube (1) holding the head (5) of the needle (3); a closing device (14) of the cavity (11) of the piston (4) at the front part of the piston (4); and appendages (10) which press on the interlocking retention devices (12) of the inside front part of the tube (1) when the piston (4) completes its operative run thereby releasing the needle (3), which, forced by the spring (6), is made to enter the cavity (11) of the piston (4) characterised in that an obturating device (7) constituted by a discoid part (7) made of a slightly flexible material is arranged across the front inside part of the tube (1) and held in an operative position by the head (5) of the needle (3); the edge of the discoid part (7) is in contact with the inside wall of the front part of the tube (1), accomplishing with a tight sealing; the discoid part (7) has a central counter-sunk hole (8) to receive the conically enlarged end (9) of the head (5) of the needle (3) passing through the hole (8) accomplising also a tight sealing between the conical surface of the end (9) of the head (5) and the surface of the countersunk hole (8); said discoid part (7) is pushed forward by the front of the piston (4) when the piston completes its operative run; said appendages (10) are arranged on the front side of the discoid part (7); and that said closing device (14) of the piston cavity (11) comprises an irreversible joining device which fits into complementary joining devices placed at the head (5) of the needle (3) intended to work together when they come into contact once the piston has completed its operative run.

2. Disposable hypodermic syringe according to claim 1, characterized in that said interlocking retention devices of the front end of the tube are made up of a plurality of flexible-tongues, arising from the wall of the tube (1) at a slanting angle, facing forward and inward, being adapted so that they become fitted into a ring-shaped external groove (13) made around the outside of the head (5) of needle (3), from where they are removed as they are bent by the pushing action of the appendages (10) of said obturating discoid part (7).

3. Disposable hypodermic syringe, according to claim 1, characterized in that said closing device (14) of the piston cavity is constituted by a discoid button, with its edge pressure-fitted into a ring-shaped groove (15) made around the mouth of the cavity of the piston (4), said discoid button having a protuberance (16) in its front part which constitutes said irreversible joining device and which has an external stepped pro-truding edge (17) around it whish is to be pressure-fitted into a countersunk hole (18) of the head (5) of needle (3) constituting said complementary joining devices, from where it is impossible to be extracted due to the opposing action of said complementary joining devices, which, in turn, consist of an indent step.

4. Disposable hypodermic syringe according to claim 1, characterized in that the piston-retaining devices (19) are arranged at the mouth of the cavity of tube (1), so that the total extraction of the piston (4) from the tube (1) is impossible.

## Patentansprüche

1. Subkutane Einwegspritze von der Art, die nach einmaligem Gebrauch nicht mehr verwendbar sind, bestehend aus einem Rohr (1), das an seinem vorderen Ende geschmälert und so eingerichtet ist, dass eine subkutane Nₐdel aufnimmt(3), deren Kopf (5) von einer Feder (6) beaufschlagt wird, die danach trachtet, die Nadel in das Innere des Rohres (1) zu drücken, aus einem hohlen Kolben, der im Inneren des Rohres (1) gleitet;Rückhaltevorrichtungen durch Einrasten(12), die frontal im Inneren des Rohres (1) liegen, um den Kopf (5) der Nadel (3) festzuhalten; eine Verschlussvorrichtung (14) der Ausnehmung (11) des Kolbens (1) an dessen Frontpartie sowie Fortsätze(10), die auf die Rückhaltevorrichtungen durch Einrastung (12) Druck ausüben im inneren Frontteil des Rohres (1), wenn der Kolben (4) seine Strecke zu Ende fährt, wodurch er die Nadel (3) freigibt, welche, gezwungen von der Feder (6) in die Ausnehmung (11) des Kolbens (4) einfährt, dadurch gekennzeichnet, dass sie mit einer Verschlussvorrichtung (7) versehen ist, die von einem scheibenförmigen Teil (7) gebildet wird, hergestellt aus einem leicht flexiblen Material, welcher den vorderen inneren Teil des Rohres (1) durchquert und durch den Kopf (5) der Nadel (3) in Funktionsstellung gehalten wird, dass der Rand des scheibenförmigen Teils (7) mit der Innenwand des vorderen Teils des Rohres (1) in Berührung steht,wodurch eine flüssigkeitsdichte Versiegelung zustande kommt;dass das scheibenförmige Teil (7) ein zentrales versenktes Loch(8) aufweist, um das kegelförmig erweiterte Ende (9) des Kopfes (5) der Nadel (3) aufzunehmen, die durch das Loch (8) kommt und ebenfalls eine flüssigkeitdichte Versiegelung zwischen der Kegelfläche des Endes (9) des Kopfes (5) und der Fläche des Senkloches (8) herstellt; dass der vordere Teil des Kolbens (4) auf besagtes scheibenförmige Teil (7) einen Schub ausübt, wenn der Kolben seine Funktionsstrecke beendet; dass die besagten Fortsätze (10) am vorderen Teil des scheibenförmigen Elements (7) angeordnet sind und dass die besagte Verschlussvorrichtung (14) der Ausnehmung des Kolbens, die vollkommen in die ergänzenden Verbindungsvorrichtungen einrastet, die sich am Kopf (5) der Nadel (3) befinden, dazu bestimmt,zusammenzuwirken, wenn sie einander in Berührung kommen, nachdem der Kolben seine Wegstrecke voll zurückgelegt hat.

2. Subkutante Einwegspritze gemäss Anspruch 1 dadurch gekennzeichnet, dass die erwähnten Rückhaltevorrichtungen durch Einrastung des vorderen Endes des Rohres von einer Mehrzahl von flexiblen Zungen gebildet werden, die aus der Wand des Rohres (1) in einem nach vorne und innen geneigten Winkel herauskommen , welche sich einpassen, da sie zu einer äusseren Rille in Form eines Ringes (13) werden, der sich aussen, rings um den Kopf (5) der Nadel (3) bildet, von wo aus sie sich zurückziehen, wenn sie durch die Schubbewegung der Fortsätze (10) des besagten scheibenförmigen Verschlussteils (7) gebogen werden.

3. Subkutane Einwegspritze gemäss Anspruch 1 dadurch gekennzeichnet, dass die besagte Verschlussvorrichtung (14) der Ausnehmung des Kolbens von einem scheibenförmigen Knopf gebildet wird, der mit seinem Rand unter Druck dem Inneren einer Furche in Form eines Ringes (15) angepasst ist, der rings um die Mundöffnung der Ausnehmung des Kolbens (4) gebildet ist,wobei der besagte Knopf in Scheibenform einen Vorsprung (16) am Frontteil aufweist, welcher die besagte unumkehrbare Verbindungsvorrichtung darstellt und der einen äusseren abgestuft hervortretenden Rand (17) aufweist, um den er sich unter Druck in das Senkloch (18) des Kopfes (5) der Nadel (3) anlegt,wobei sie die genannten komplementären Verbindungselemente darstellen, aus denen er nicht mehr herausgezogen werden kann aufgrund der entgegengesetzen Wirkung dieser komplementären Verbindungsvorrichtungen, die ihrerseits eine gezahnte Stufe bilden.

4. Subkutane Einwegspritze gemäss Anspruch 1 dadurch gekennzeichnet, dass die Rückhaltevorrichtungen des Kolbens (19) an der Mundöffnung der Ausnehmung des Rohres (1) vorgesehen sind sodass ein völliges Herausziehen des Kolbens (4) aus dem Rohr (1) unmöglich wird.

## Revendications

1. Seringue hypodermique rejetable du genre devenant inutilisable après s'en être servi, qui comprend un tube (1) qui est plus mince et adapté dans sa partie frontale pour recevoir une seringue hypodermique (3), dont la tête (5) est pressée par un ressort (6) tendant à la pousser vers l'intérieur du tube (1); un piston creux (4) qui glisse par la partie intérieure du tube (1); des dispositifs de retenue par enclavement (12) placés dans la partie intérieure frontale du tube (1) pour retenir la tête (5) de l'aiguille (3); un dispositif de fermeture (14) de la cavité (11) du piston (4) dans la partie frontale du piston (4); et des appendices (10) faisant pression sur les dispositifs de retenue par enclavement (12) de la partie intérieure frontale du tube (1) quand le piston (4) complète son parcours opérationnel, et libère l'aiguille (3) qui, obligée par le ressort (6), s'introduit dans la cavité (11) du piston (4), qui se définit par le fait de porter un dispositif d'obturation (7) constitué par une pièce en forme de disque (7) fabriquée en un matériel légèrement souple, qui traverse la partie intérieure frontale du tube (1) et maintenu en position opérationnelle par la tête (5) de l'aiguille (3); le bord de la pièce en forme de disque (7) est en contact avec la paroi intérieure de la partie frontale du tube (7), ce qui permet d'effectuer un scellage étanche; la partie en forme de disque (7) a un orifice central en forme d'amende (8) pour recevoir l'extrême coniquement élargi (9) de la tête (5) de l'aiguille (3), qui passe à travers l'orifice (8) et effectue aussi un scellage étanche entre la surface conique de l'extrême (9) de la tête (5) et la surface de l'orifice en forme d'amande (8); la partie frontale du piston (4) pousse cette partie en forme de disque (7) quand le piston complète son parcours opératif; les appendices mentionnés (10) sont disposés dans la partie frontale de la pièce en forme de disque (7); et le dispositif de fermeture mentionné (14) de la cavité du piston, qui case complètement dans les dispositifs d'union complémentaires situés dans la tête (5) de l'aiguille (3) dont le but est de travailler ensemble quand ils entrent en contact, une fois que le piston a complété sa carrière opérative.

2. Seringue hypodermique rejetable, d'accord avec la reivindication 1, définie par le fait que de tels dispositifs de retenue par enclavement de l'extrême frontal du tube sont formés par une pluralité de pattes souples sortant de la paroi du tube (1) en un angle incliné en avant et vers l'intérieur, étant adaptés pour devenir une cannelure externe en forme d'anneau (13) qui se forme autour de l'extérieur de la tête (5) de l'aiguille (3), d'où ils se retirent, car ils sont pliés par l'action de pression des appendices (10) de cette partie obturatrice en forme de disque (7).

3. Seringue hypodermique rejetable, selon la reivindication 1, définie par le fait que le dispositif de fermeture mentionné (14) de la cavité du piston est formé par un bouton en forme de disque avec bord ajusté à pression à l'intérieur d'un sillon en forme d'anneau (15), formé autour de la bouche de la cavité du piston (4), ayant ce bouton en forme de disque une protubérance (16) dans sa partie frontale, qui est le dispositif d'union irréversible, et qui a un bord extérieur protubérant échelonné (17) autour duquel s'ajuste à pression dans l'orifice en forme d'amende (18) de la tête) de l'aiguille (3), devenant partie de ces dispositifs complémentaires d'union desquels ils ne peut être extrait à cause de l'action opposée de ces dispositifs complémentaires d'union qui, à leur tour, forment un échelon crénelé.

4. Seringue hypodermique rejetable d'accord avec la reivindication 1, définie par le fait que les dispositifs de retenue du piston (19) sont placés dans la bouche de la cavité du tube (1) de manière telle que l'extraction totale du piston (4) du tube (1) en est impossible (7).
